# EUROPEAN PATENT SPECIFICATION

(11) **EP 4 295 825 B1**
(45) Date of publication and mention of the grant of the patent: **04.11.2026**
(21) Application number: 22179838.2
(22) Date of filing: 20.06.2022
(51) Int. Cl.: A61F 11/14, H04R 1/1025, H04R 5/033

(54) **HEARING PROTECTOR WITH PHOTOVOLTAIC ELEMENT AND METHOD OF RETROFITTING A HEARING PROTECTOR THEREWITH**
GEHÖRSCHUTZ MIT PHOTOVOLTAISCHEM ELEMENT UND VERFAHREN ZUR NACHRÜSTUNG EINES GEHÖRSCHUTZES DAMIT
DISPOSITIF DE PROTECTION AUDITIVE AVEC ÉLÉMENT PHOTOVOLTAÏQUE ET PROCÉDÉ DE MODERNISATION D'UN DISPOSITIF DE PROTECTION AUDITIVE COMPORTANT CELUI-CI

(43) Date of publication of application: 27.12.2023
(73) Proprietor: 3M Innovative Properties Company, Saint Paul, MN 55133-3427 (US)
(72) Inventor: Hjertberg, Tim, 33102 Värnamo (SE); Karlsson, Robert, 33102 Värnamo (SE); Hjort, Patrick, 33102 Värnamo (SE); Nilsson, Jonas, 33102 Värnamo (SE)
(74) Representative: Mathys & Squire

(56) References cited:
- WO-A1-2014/060729
- WO-A1-2018/002883
- WO-A2-02/01910
- DE-U1- 202009 001 941
- US-A1- 2019 373 353

## Description

The present disclosure relates to a hearing protector with a photovoltaic element and to a method of retrofitting a hearing protector with a photovoltaic element.

Hearing protectors as personal protection equipment (PPE) are typically used in noisy environments for protecting a wearer's hearing from noise at potentially harmful noise levels. Typically, hearing protectors have two earmuffs or cups which cover the ears of the wearer or user and which are connected to one another by a headband. Each cup further typically is formed by a rigid outer shell that is furnished with a sound absorbing or noise dampening material, for example a foamed material, for reducing unwanted ambient sound. In addition to passive noise reduction or cancellation, the hearing protector may comprise an active noise reduction or cancellation using a technology where the ambient sound is captured by a microphone and inverted sounds waves, i.e. sound waves being exactly out of phase, are being generated and played via loudspeakers in the headset to the user in parallel to the ambient noise reaching the user's ears. Typically, these opposite sounds waves collide and eliminate each other or cancel each other. Hearing protectors may also comprise other active components, e.g. an active communication device comprising a loudspeaker and electronic circuitry, wherein the electronic circuitry may be configured to drive the loudspeaker. Such active components may also include a wireless communication interface and a microphone for enabling voice communication. Furthermore, sensors to obtain information about the surrounding of the hearing protector and/or of the user thereof are also conceivable as active components. Such hearing protectors may, for example, be described in WO 2018/002883 A or WO 2019/104172 A. Hearing protectors may be worn over an extended period of time and may thus need a sufficiently long operating time. Typically, the electric energy for operating the active components is provided by batteries, which may be of the single-use type or which may be rechargeable. The battery life is therefore important for the operating time and function of the hearing protector, in particular as some of the aforementioned active components may be relevant for the safety of the user, e.g. voice communication or gathering ambient data by sensors.

DE 202009001941 U1 discloses a hearing protection device comprising two ear protection cups connected by a headband, wherein at least one ear protection cup comprises an active component. The hearing protection device further comprises a solar cell for powering the active component, the solar cell being connected to the active component via an energy supply including rechargeable batteries.

WO 2018/002883 A1 discloses a method of retrofitting a hearing protector comprising two earmuffs and a headband with an additional element, wherein at least one of the earmuffs comprises an active component. The method comprises providing the hearing protector, providing the additional element and attaching the additional element to the hearing protector, wherein the earmuffs each comprise a cushion, an outer shell defining a hollow space therein and sound absorbing material.

Thus, there is a need to improve the hearing protector such that the function is provided for a sufficiently long time period, in particular to improve the operating time of the hearing protector.

In a first aspect, the present disclosure relates to a hearing protector as defined in claim 1. Particularly, the hearing protector comprises two earmuffs each comprising a cushion, an outer shell defining a hollow space therein and sound absorbing material. The hearing protector comprises a headband for retaining the earmuffs in position when worn by a user. At least one of the earmuffs comprises an active component. The hearing protector comprises a photovoltaic element for powering the active component. The photovoltaic element is attached to the headband. The photovoltaic element is electrically connected to the active component of the earmuff. The advantage of such a hearing protector is that the photovoltaic element provides the electric energy necessary for operating the active component of the hearing protector. Depending on the light conditions and the provision of electric energy by the photovoltaic element, the energy supply of the active component may partially or entirely be independent of a battery which may be present at the hearing protector and/or extend the operating time such that the active component can be operated for a longer time. Also, an extension of the battery life and a re-charging of the battery may be achieved by such a hearing protector, in particular if the hearing protector is not in use or even in use depending on the light conditions and the yield of electric energy from the photovoltaic element. An increase in safety for the user may also be achieved thereby, in particular if the active components of the hearing protector are safety relevant such as voice communication and/or gathering ambient data through sensors.

In a second aspect, the present disclosure relates to a method of retrofitting a hearing protector with a photovoltaic element as defined in claim 12. The hearing protector comprises two earmuffs and a headband for retaining the earmuffs in position when worn by a user. At least one of the earmuffs comprises an active component. The method comprising the steps of providing a hearing protector comprising two earmuffs and a headband for retaining the earmuffs in position when worn by a user, wherein at least one of the earmuffs comprises an active component, providing a elongate photovoltaic element, attaching the photovoltaic element to the headband, electrically connecting the photovoltaic element to the active component of the hearing protector. The earmuffs each comprises a cushion, an outer shell defining a hollow space therein and sound absorbing material. The benefit of such a method of retrofitting is that a hearing protector initially not having a photovoltaic element is provided therewith which enables the hearing protector to have an improved supply of electric energy as described above for the hearing protector according to the first aspect of the present disclosure, in particular to provide electric energy from the photovoltaic element to the active component of the hearing protector and to prolong the operating time and battery life of a battery present at a hearing protector, respectively. The so retrofitted hearing protector enjoys the same advantages as described above for the hearing protector according to the first aspect of the present disclosure.

Hearing protectors typically have two muffs or caps which cover the ears of the wearer and which are connected to one another by a headband. Each cup further typically is formed by a rigid outer shell that is furnished with a sound absorbing or noise dampening material, for example a foamed material. Each cup has an ear-facing side, which - in use - faces towards the ear of a user and a closed side, which faces away from the user's ear. Typically, materials for the cushion comprise a porous or foamed material. Alternatively, the materials comprise a non-porous or foam-free material, e.g. silicone material of a theoretical density of between 1.1 g/cm³ and 1.2 g/cm³. The material and the structure of the cushion typically provides for resilient properties allowing the cushion to adapt tightly and sealingly with a user's or wearer's skin. It is also conceivable to include rubber as material for the cushion. The cushion may be cut-off or molded to achieve the desired size and shape.

Active or passive noise reduction or cancellation within the meaning of the present disclosure is understood as measures in a hearing protector that reduces unwanted ambient sound or noise. This may be achieved by passive measures, e.g. where the cups and/or cushions of the headset comprise sound absorbing or noise damping material and wherein the cushions cover the user's ears, i.e. passive noise cancellation may be achieved by the use of sound absorbing or noise damping material which prevents the conducting of noise into the earmuff. This includes noise absorption as well. Alternatively or in addition to passive noise reduction or cancellation, active noise reduction or cancellation uses a technology where the ambient sound is captured by a microphone and inverted sounds waves, i.e. sound waves being exactly out of phase, are being generated and played via loudspeakers in the hearing protector to the user in parallel to the ambient noise reaching the user's ears. Typically, these opposite sounds waves collide and eliminate each other or cancel each other.

Sound absorbing or noise damping material within the meaning of the present disclosure is understood as material that limits the propagation of frequencies into a space, i.e. help to reduce sound or noise that enters a space, e.g. the earmuff of a hearing protector. Sound absorbing or noise damping material is able to take the energy from the sound and turns it into another type of energy. Sound absorbing or noise damping material may include dense, but soft materials to absorb sound or vibration waves hitting the material. It is noted that the terms sound absorbing material and noise damping materials are used similarly throughout this description. Sound absorbing or noise damping materials include porous or non-porous materials, e.g. open-cell foams, closed-cell foams, open-cell foams with a sealed outer layer or non-porous materials such as silicone, thermoplastic elastomer (TPE) or thermoplastic urethane (TPU).

An active component within the meaning of the present disclosure may include an active noise cancellation as described above or a sensor as described below. Furthermore, active components may include a communication device comprising a loudspeaker and electronic circuitry which comprises a wireless communication interface, the electronic circuitry being configured to drive the loudspeaker based on information received via the wireless communication interface. Such an active communication device is useful as it enables the user or wearer of such a hearing protector to communicate with others in the surrounding even without taking off the hearing protector. Thereby, a risk of taking off the hearing protector because of the need to communicate in a noisy environment is reduced or avoided.

Sensors suitable for the hearing protector according to the present disclosure comprise cushion sensors for sensing conditions of the cushion of the skin contacted by the cushion, cup sensors for sensing conditions of the cup, skin sensors for sensing skin conditions of a user or wearer, physiological sensors for sensing physiological parameters of a user or wearer, environmental sensors for sensing environmental or ambient conditions of the surrounding of a user or wearer and/or movement sensors for sensing a movement of a user or wearer.

A photovoltaic element within the meaning of the present disclosure is an element for conversion of light into electricity using semiconductor materials exhibiting the photovoltaic effect. Typically, a photovoltaic element includes a solar cell or a solar module comprising a number of solar cells, respectively. The solar cell generates electrical power. The photovoltaic element may also comprise an edge region surrounding the solar cell which may be used for mounting the photovoltaic element to a structure. Typically, the photovoltaic element is flat.

In one embodiment, the photovoltaic element of the hearing protector is attached to the headband by a fixation frame. The fixation frame covers the entire perimeter of the photovoltaic element. The advantage of such a fixation frame, in particular covering the perimeter, is that an easy and reliable way for attaching the photovoltaic element is provided thereby. Also, such an arrangement may allow for an optional sealing to be arranged at the fixation frame and/or provided by the fixation frame.

In a further embodiment, the headband of the hearing protector comprises rods or wires. The rods or wires may provide stability and flexibility to the headband. Also, the earmuffs extensions of the headband may be formed thereby. The rods or wires may be made of metal or a polymeric material and may exhibit a spring-like behavior. The rods or wires represent an easy and reliable way of providing the required properties to the headband of the hearing protector, i.e. conformability of the hearing protector to the user's head with a sufficient contact pressure.

In another embodiment, the fixation frame of the hearing protector is a molded part attached to the headband by overmolding. With overmolding, it is meant that the molding process provides the fixation frame and the attachment at the same time. The advantage of such a molded part is that an easy and reliable way of making the fixation frame is provided thereby.

In a further embodiment, the molded fixation frame of the hearing protector is attached to the headband by chemical bonding, preferably by means of an adhesive. Such an arrangement allows for more design freedom as different materials could be used.

The hearing protector according to the present disclosure is made by providing a hearing protector having two earmuffs and a headband for carrying the earmuffs. A photovoltaic element of a flat, elongate shape is then provided to be mounted to the headband by suitable methods such as molding. This step may include the provision of a support element with a honeycomb structure onto which the photovoltaic element is placed and which is then overmolded to create a fixation frame for fixing the photovoltaic element at the support structure and the headband, respectively. It is noted that overmolding means that the molding of the fixation frame and the fixing of the photovoltaic element happens at a time. Other way of attaching or fixing of the photovoltaic element to the support structure and the headband, respectively, are conceivable. The photovoltaic element is then electrically connected to the active components of the hearing protector which are located in one or both earmuffs by an electric cable, preferably the electric cable for carrying signals between the active components of the earmuffs. The electric cable may comprise several wires to allow for signal transmission as well as to allow energy transmission therewith. It is noted that the flat elongate photovoltaic element is brought from a flat condition to a curved condition generally conforming to the shape of the headband of the hearing protector.

In a further embodiment, the fixation frame of the hearing protector comprises a sealing along the perimeter of the fixation frame. Such a sealing may, for example, comprise flexible or compressible material suitable for sealings such as rubber applied onto the edge region of the photovoltaic element. Alternatively, the sealing may be provided by the molded part, i.e. the fixation frame, adhering to the edge region of the photovoltaic element. It is conceivable that the sealing is arranged with a distance to the perimeter, e.g. 1 to 10 mm. The advantage of such a sealing is that the ingress of water, dust or dirt is reduced or prevented thereby. This is particularly important as the photovoltaic element is attached to the headband and has portions exposed to the above, i.e. to falling rain, particles or the like. The sealing may also provide some stability to the photovoltaic element attached to the headband. It is noted that the sealing may exhibit adhesive properties, e.g. through the molding onto the photovoltaic element resulting in adhesion between the surface of the photovoltaic element and the molded part. The sealing may also be provided by an adhesive applied between the fixation frame and the photovoltaic element. Furthermore, a sealing as described above, e.g. made of rubber, may also exhibit adhesion, e.g. due to a coating with an adhesive.

In yet another embodiment, wherein the fixation frame of the hearing protector covers not more than 12%, preferably not more than 10%, more preferred not more than 7,5% of the surface of the photovoltaic element. Such an arrangement is beneficial as it provides for a good balance between reliable attachment of the photovoltaic element to the headband of the hearing protector on the one hand and not covering too much of the surface area of the photovoltaic element on the other hand which may otherwise lead to a reduced electric energy yield.

In yet a further embodiment, the headband of the hearing protector comprises a support element having a honeycomb structure. The support element may be arranged on the inner side of the headband, i.e. the side facing - in use - towards the head of a wearer or user of the hearing protector or the outer side, i.e. the side facing - in use - away from the user's head. The support element may be attached to the headband by suitable attachment means including mechanical attachment means such a clips, clamps, hooks or the like or by an adhesive such as an adhesive layer or adhesive tape. It is noted that the support element may exhibit an elastic behavior such that it conforms to the headband and such that it follows movements of the headband, e.g. bending or the like during donning or doffing of the hearing protector. The advantage of such a support element is that the headband is provided with sufficient stability and resistance against bending thereby protecting the photovoltaic element attached thereto. A honeycomb structure of the support element provides for an easy and reliable structure while maintain a lightweight construction of the support element, headband and hearing protector. In certain embodiments, the honeycomb structure of the support element of the hearing protector exhibits an elongate shape, wherein the honeycomb structure is preferably molded. An elongate shape is beneficial as such a shape would be similar to the elongate shape of the headband to which the support element is attached to. Molding of the honeycomb structure of the support element represents an easy, cost-efficient and reliable way of providing such an element. In case of molding of the honeycomb structure, the rods or wires of the headband, if present, may be encapsulated or encompassed by the molded part to facilitate attachment. In certain embodiments, the honeycomb structure of the support element of the hearing protector comprises ribs, preferably defining hollow spaces therebetween. Preferably, the ribs are arranged in a pattern. Such ribs are advantageous as these provide for a sufficient stability while maintaining a lightweight structure and while easy to manufacture. In certain embodiments, the honeycomb structure of the support element of the hearing protector comprises ribs in a direction along the elongate extension of the honeycomb structure and ribs perpendicular to the elongate extension. Such oriented ribs provide a good stability and are easy to manufacture, for example by molding. In certain embodiments, the ribs of the honeycomb structure of the support element are arranged in parallel with each other. Such orientation provides for a good stability of the support element while easy to manufacture. In certain embodiments, the ribs are evenly spaced from each other. Such an orientation is easy to manufacture while maintaining good stability of the support element. In certain embodiments, the ribs of the honeycomb structure of the support element are separated by a spacing of 1 to 20 mm, preferably of 3 to 8 mm. Such a spacing provides for a good balance of stability and lightweight structure. Also, a good conformability may be achieved thereby. It is noted that the ribs may exhibit a curved shape because the ribs and the support element, respectively, are attached to the headband which also exhibits a curved shape.

In one embodiment, the active component of the hearing protector is arranged in the hollow space of the earmuff. Such an arrangement is beneficial as there is no additional space required and no parts of the active component stick out. A protection of the active component by the earmuff may also be achieved thereby.

In a further embodiment, the photovoltaic element of the hearing protector exhibits an elongated shape. An elongate shape is beneficial as such a shape would be similar to the elongate shape of the headband to which the photovoltaic element is attached.

In yet a further embodiment, the photovoltaic element of the hearing protector is preshaped exhibiting a curved shape. Such a photovoltaic element is beneficial as it sufficiently conforms to the headband exhibiting a curved shape to which the photovoltaic element is attached to.

According to the invention, the photovoltaic element of the hearing protector is flexible. This ensures that the photovoltaic element would follow movements of the headband to which the photovoltaic element is attached which may occur when the hearing protector is being used including donning or doffing thereof. It is noted that the photovoltaic element may have an initial flat shape which is beneficial for storage and shipment. The curved shape of the photovoltaic element is a result of the flexibility and the attachment to the headband of the hearing protector exhibiting a curved shape, respectively.

In yet another embodiment, the hearing protector comprises a rechargeable battery, wherein the rechargeable battery is electrically connected to the photovoltaic element. Such a rechargeable battery provides for more flexibility in use of the hearing protector as the rechargeable battery may be charged by the electric energy provided by the photovoltaic element and the stored electric energy may be used at a later time, e.g. when there is no energy yield from the photovoltaic element due to insufficient light conditions.

In a further embodiment, each earmuff of the hearing protector comprises an active component. The hearing protector further comprises a signal cable for connecting the active components of each of the earmuffs and wherein the electric power for powering the active component is distributed over the signal cable. Typically, the signal cable is an electric cable comprising several wires or conductors which are arranged within a common cable insulation. The signal distribution and the electric power distribution may be through the same wires or conductors. It is also conceivable, that the distribution of the signals and the electric power is through different wires or conductors. The advantage of distributing the electric power over the signal cable is that only one cable needs to be arranged, which simplifies the construction of the hearing protector.

In still another embodiment, the sound absorbing material of the earmuff of the hearing protector comprises porous or non-porous materials, e.g. open-cell foams, closed-cell foams, open-cell foams with a sealed outer layer or non-porous materials such as silicone, thermoplastic elastomer (TPE) or thermoplastic urethane (TPU). Such a material is of advantage because a reliable and cost-efficient way of sound absorption is achieved thereby.

In a further embodiment, the hearing protector exhibits a minimum sound attenuation M of at least 11 dB according to EN352-1:2020 or according to EN352-3:2020. Such a noise attenuation is of advantage because a sufficient and reliable hearing protection is provided thereby.

In yet a further embodiment, in the method of retrofitting a hearing protector with a photovoltaic element, the headband comprises a support element having a honeycomb structure. The support element may be arranged on the inner side of the headband, i.e. the side facing - in use - towards the head of a wearer or user of the hearing protector or the outer side, i.e. the side facing - in use - away from the user's head. The support element may be attached to the headband by suitable attachment means including mechanical attachment means such as clips, clamps, hooks or the like or by an adhesive such as an adhesive layer or adhesive tape. It is noted that the support element may exhibit an elastic behavior such that it conforms to the headband and such that it follows movements of the headband, e.g. bending or the like during donning or doffing of the hearing protector. The advantage of such a support element is that the headband is provided with sufficient stability and resistance against bending thereby protecting the photovoltaic element attached thereto. A honeycomb structure of the support element provides for an easy and reliable structure while maintain a lightweight construction of the support element, headband and hearing protector. In certain embodiments, the honeycomb structure of the support element of the hearing protector exhibits an elongate shape, wherein the honeycomb structure is preferably molded. An elongate shape is beneficial as such a shape would be similar to the elongate shape of the headband to which the support element is attached to. Molding of the honeycomb structure of the support element represents an easy, cost-efficient and reliable way of providing such an element. In case of molding of the honeycomb structure, the rods or wires of the headband, if present, may be encapsulated or encompassed by the molded part to facilitate attachment of the support element to the headband. In certain embodiments, the honeycomb structure of the support element of the hearing protector comprises ribs, preferably defining hollow spaces therebetween. Preferably, the ribs are arranged in a pattern. Such ribs are advantageous as these provide for a sufficient stability while maintaining a lightweight structure and while easy to manufacture. In certain embodiments, the honeycomb structure of the support element of the hearing protector comprises ribs in a direction along the elongate extension of the honeycomb structure and ribs perpendicular to the elongate extension. Such oriented ribs provide a good stability and are easy to manufacture, for example by molding. In certain embodiments, the ribs of the honeycomb structure of the support element are arranged in parallel with each other. Such orientation provides for a good stability of the support element while easy to manufacture. In certain embodiments, the ribs are evenly spaced from each other. Such an orientation is easy to manufacture while maintaining good stability of the support element. In certain embodiments, the ribs of the honeycomb structure of the support element are separated by a spacing of 1 to 20 mm, preferably of 3 to 8 mm. Such a spacing provides for a good balance of stability and lightweight structure. Also, a good conformability may be achieved thereby. It is noted that the ribs may exhibit a curved shape because the ribs and the support element, respectively, are attached to the headband which also exhibits a curved shape.

In still a further certain embodiment, in the method of retrofitting a hearing protector with a photovoltaic element, the honeycomb structure exhibits an elongate shape and wherein the honeycomb structure is preferably molded. An elongate shape is beneficial as such a shape would be similar to the elongate shape of the headband to which the support element is attached. Molding of the honeycomb structure of the support element represents an easy, cost-efficient and reliable way of providing such an element.

In another embodiment, in the method of retrofitting a hearing protector with a photovoltaic element, the honeycomb structure comprises ribs, preferably defining hollow spaces formed therebetween. Preferably, the ribs are arranged in a pattern. Such ribs are advantageous as these provide for a sufficient stability while maintaining a lightweight structure and while easy to manufacture.

In yet another certain embodiment, in the method of retrofitting a hearing protector with a photovoltaic element, the step of attaching the photovoltaic element comprises molding such that a fixation frame is formed for fixing the photovoltaic element at the headband. The advantage of such a molded part is that an easy and reliable way of making the fixation frame is provided thereby.

The invention was described in various embodiments above. It is understood by a person skilled in the art, that one of, several of or all the above-mentioned embodiments can be combined with each other.

The invention will now be described in more detail with reference to the following Figures exemplifying particular embodiments of the invention:

### Brief description of the Figures:

Fig. 1 is a schematic front view of the hearing protector according to an embodiment of the present disclosure worn by a user;
Fig. 2 is a schematic functional front view of the hearing protector according to an embodiment of the present disclosure;
Fig. 3 is a schematic perspective exploded view of the hearing protector according to an embodiment of the present disclosure;
Fig. 4 is a schematic perspective view of the hearing protector according to an embodiment of the present disclosure;
Fig. 5 is a schematic cross-sectional view of a detail of the hearing protector according to an embodiment of the present disclosure as shown in Fig. 2;
Fig. 6 is a schematic top view of a detail of the hearing protector according to an embodiment of the present disclosure as shown in Fig. 3; and
Fig. 7 is a schematic perspective view of a detail of the hearing protector according to an embodiment of the present disclosure as shown in Fig. 3.

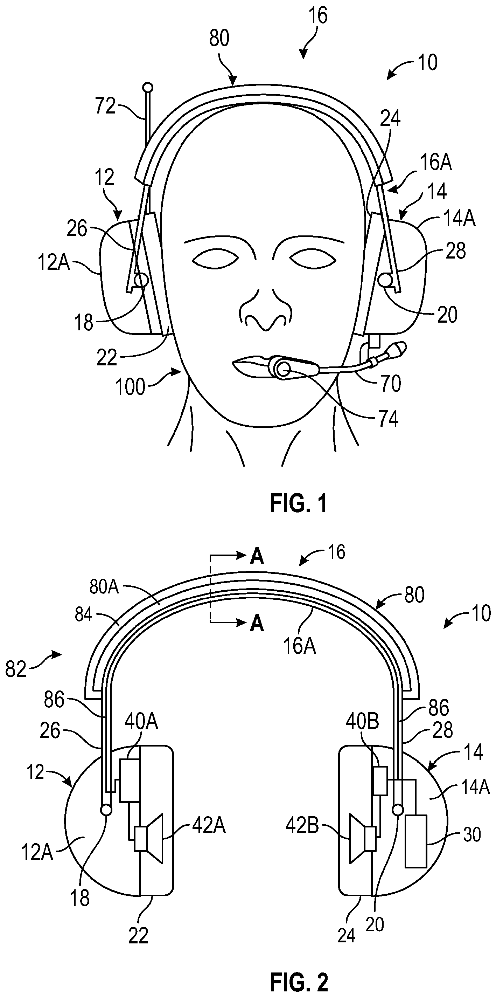

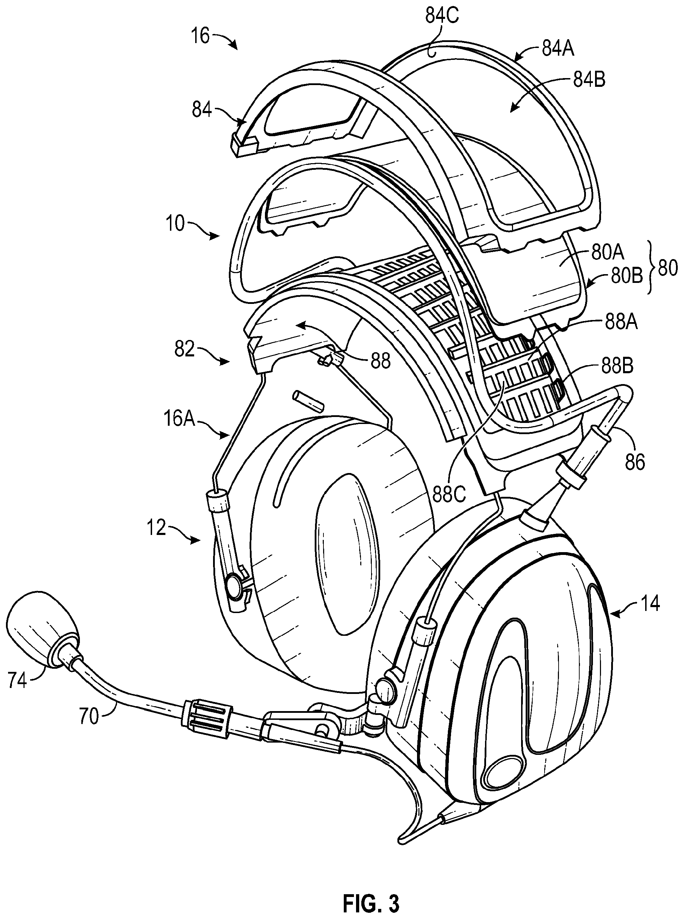

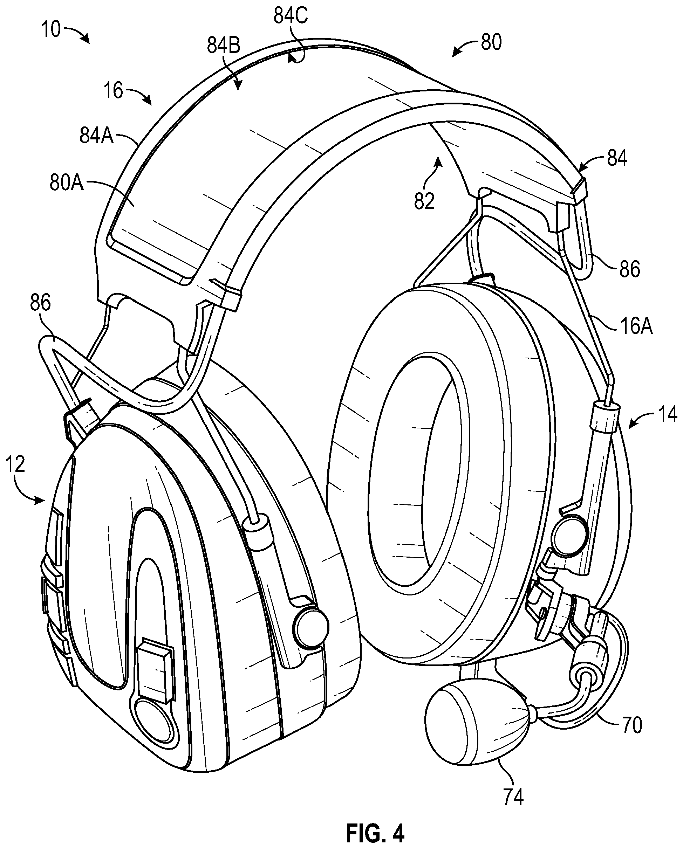

Fig. 1 shows in a schematic front view of the hearing protector 10 according to an embodiment of the present disclosure worn by a user 100. As can be seen from Fig. 1, the hearing protector 10 comprises two earmuffs 12, 14 each having an outer shell or cup 12A, 14A. Attached to each of the earmuffs 12, 14 are cushions 22, 24 to contact the skin of the user 100 for providing a tight seal against the environment of the hearing protector 10, e.g. against ambient noise. Each of the earmuffs 12, 14 is mounted to a headband 16 by earmuff mounts 18, 20 engaging with the earmuff mounts of the headband and with the earmuff extensions 26, 28 of the headband 16, respectively, which keep the earmuffs 12, 14 connected to the headband in a pivoting relation to adapt to the user's skin. The headband 16 comprises rods or wires 16A providing stability and flexibility to the headband 16. The rods or wires 16A may be made of metal and may exhibit a spring-like behavior. The above-mentioned earmuff extensions 26, 28 of the headband 16 may be provided by the rods or wires 16A. The hearing protector 10 comprises a photovoltaic element 80 attached to the headband 16, which is only schematically visible here (for more details, see Figs. 3 and 4). One or both outer shells or cups 12, 14 of the hearing protector 10 comprise an active component 40A, 40B, which has been omitted in Fig. 1 (please see Fig. 2). The hearing protector 10 may further comprise - as part of an active communication system - a microphone 74 mounted to one of the earmuffs 14 by a microphone boom 70. The microphone 74 enables the user 100 to send voice signals for communication to other users in the surrounding. The hearing protector 10 may also comprise an antenna 72 as part of a communication system to send and/or receive signals incl. data, e.g. about ambient conditions, messages and/or voice signals. It is noted that the microphone 74 with its microphone boom 70 and/or the antenna 72 may extend from the other earmuff 12, 14 different to as it is shown in Fig. 1. Also, it is conceivable that two antennas 72 are arranged, i.e. one from each of the earmuffs 12, 14.

Fig. 2 is a schematic cross-sectional view of the hearing protector 10 according to an embodiment of the present disclosure. The hearing protector 10 comprises two earmuffs 12, 14 connected and carried by a headband 16. The earmuffs 12, 14 each comprise an earmuff mount 18, 20 through which each of the earmuffs 12, 14 is attached to corresponding earmuff attachment means of the headband 16 (not visible in the schematic view of Fig. 2). The earmuffs 12, 14 are carried thereby by the headband 16. Similar to the hearing protector 10 as shown in Fig. 1, the hearing protector 10 of Fig. 2 comprises rods or wires 16A at the headband 16 (for details of the rods or wires 16A, please see Fig. 1). The earmuffs 12, 14 each comprise a cup 12A, 14A defining a hollow space therein. In the hollow space of one or both earmuffs 12, 14, active components 40A, 40B of the hearing protector 10 may be housed, for example printed circuit boards with electronic components mounted thereon, components of an active communication unit or sensors including microphones for sensing ambient conditions including sound. It is however conceivable that both earmuffs 12, 14 comprise parts which are only illustrated for one earmuff 12, 14 in Fig. 2. The earmuffs 12, 14 may also comprise, for example, an amplifier and a loudspeaker 42A, 42B as part of an active noise cancelling unit and/or as part of an active communication unit. Each of the cups or outer shells 12, 14A, respectively, of the earmuffs 12, 14 have a cushion 22, 24 attached thereto. The cushion 22, 24 comprises a soft, conformable and/or compressible material thereby sealing the earmuffs 12, 14 to the skin of a wearer 100 (not shown in Fig. 2, see Fig. 1). The open sides of the earmuffs, 12, 14 and the outer shells or cups 12A, 14A and the cushions 22, 24 are preferably ring-shaped and are preferably shaped and sized essentially in accordance with each other such that an easy and reliable connection is achieved between these. The cushions 22, 24 may comprise attachment means, e. g. adhesives or mechanical attachment means, which are not shown here, to facilitate the attachment to the outer shell or cup 12A, 14A. Additionally, the hearing protector 10 comprises a flat, elongate photovoltaic element indicated with 80 which is only schematically shown in Fig. 2 (for more details, please see Figs. 3 and 4). The photovoltaic element 80 is attached to the headband 16 by a fixation frame indicated with 84 which is also only schematically visible here (for details, please see Figs. 3 and 4). The photovoltaic element 80 comprises a solar cell 80A for generating electric energy from incident light. The hearing protector 10 also comprises a support element 82 which is only schematically visible here (for more details, please see Figs. 3 and 4). One of the earmuffs 14 comprise a battery 30 for powering the active components 40A, 40B. It is noted that the battery 30, although shown as located in earmuff 14, also is configured to power the active components 40A, 40B of the other earmuff 12, e.g. by arranging a power distribution cable (not shown) between the two earmuffs 12, 14. The battery 30 may be rechargeable and may store electric energy provided by the photovoltaic element 80 so that the electric energy can be used at a later time, e.g. when there is no energy yield from the photovoltaic element 80 due to insufficient light conditions. Although only shown for one of the earmuffs 14, it is also conceivable that both earmuffs 12, 14 comprise a battery 30, which may be rechargeable. The hearing protector 10 also comprises - similar to the embodiment as shown in Fig. 1 - a microphone 74 carried by a microphone boom 70 extending from the earmuff 14 and an antenna 72 (the latter of which has been omitted in Fig. 2) as part of an active communication system for transferring data and/or voice signals.

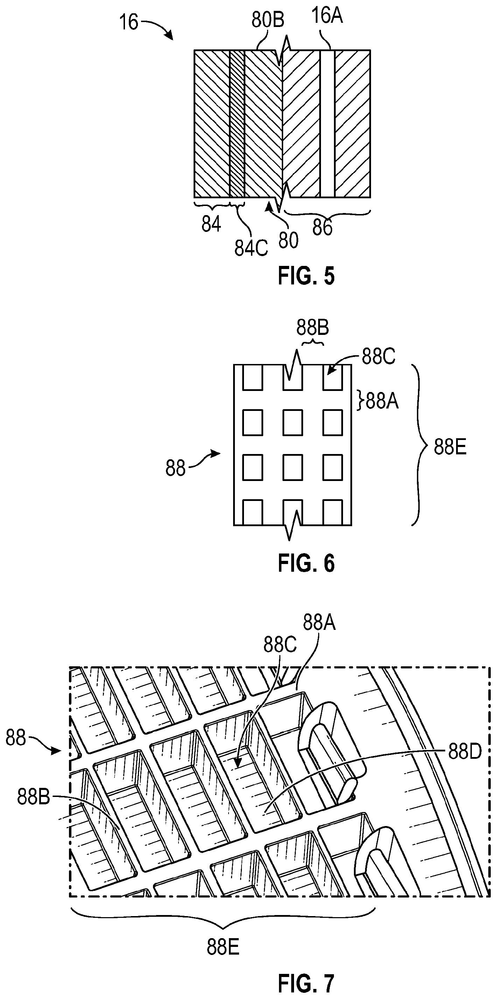

Fig. 3 shows in a schematic perspective exploded view the hearing protector 10 according to an embodiment of the present disclosure. The hearing protector 10 is similar to the hearing protector 10 as shown in Figs. 1 and 2 and comprises two earmuffs 12, 14 carried by a headband 16. Similar to the hearing protectors 10 as shown in Figs. 1 or 2, the hearing protector 10 as shown here comprises rods or wires 16A which are similar in shape and function as described above. Attached to the headband 16 is the photovoltaic element 80 comprising a solar cell 80A for generating electric energy from incident light and an edge region 80B. As can be seen, the photovoltaic element 80 is attached to the headband 16 by a fixation frame 84 which holds the photovoltaic element in a curved shape conforming to the curved shape of the headband 16. The fixation frame 84 comprises a cover 84A which mainly covers the edge region 80B and to some minor extent the solar cell 80A of the photovoltaic element 80. An opening 84B is formed in the fixation frame 84 surrounded by the cover 84A such that the main area solar cell 80A of the photovoltaic element 80 is exposed to incident light. The cover 84A houses the electric cable 86 which electrically connects the active components 40A, 40B (not shown here) of the two earmuffs 12, 14 with each other. The electric cable 86 also connects the solar cell 80A of the photovoltaic element 80 with the active components 40A, 40B of the two earmuffs 12, 14 on the one hand and with the battery 30 which may be present in one or both earmuffs 12, 14 on the other hand such that electric energy yielded by the photovoltaic element 80 can be stored in the battery 30. The electric cable 86 may also connect the active components 40A, 40B with the battery 30, if present. It is noted that the electric cable 86 may comprise several wires to allow for signal transmission as well as to allow energy transmission therewith. The cover 84A is attached to the honeycomb structure 88 of the support element 82 of the headband 16 thereby encompassing or encapsulating - together with the support element 82 and the fixation frame 84 - the headband 16 and its rods or wires 16A which may be made of metal and may have a spring-like behavior. The honeycomb structure 88 of the support element 82 comprises ribs 88A, 88B which are arranged in a pattern 88E of ribs 88A, 88B. In the example shown, the ribs 88A are arranged in parallel to each other and perpendicular to the ribs 88B, which are in turn arranged in parallel to each other. It is noted that the fixation frame 84 with its cover 84A, the support element 82 and the honeycomb structure 88 are in a curved shape similar to the curved shape of the headband 16. It is further noted that the ribs 88A, 88B also exhibit a curved shape here. The ribs 88A, 88B define an opening or space therebetween which is indicated with 88C in Fig. 3. The honeycomb structure 88 may comprise a base 88D (not shown here, see Fig. 7) which delimits the opening or space 88C on one side thereof, preferably the lower side oriented towards the user's head. Similar to the embodiments as shown in Figs. 1 and 2, the hearing protector 10 comprises a microphone 74 carried by a microphone boom 70 extending from one earmuff 14 and an antenna 72 (the latter of which has been omitted here).

Fig. 4 shows in a schematic perspective view the hearing protector 10 according to an embodiment as shown in Fig. 3. Similar to Fig. 3, the hearing protector 10 comprises two earmuffs 12, 14 carried by the headband 16. The hearing protector 10 further comprises a microphone 74 carried by a microphone boom 70 extending from the earmuff 14 and may comprise an antenna 72 (not shown here). The photovoltaic element 80 of the hearing protector 10 comprises a solar cell 80A attached to the headband 16 by the fixation frame 84. It is noted that the headband 16 of the hearing protector 10 as shown in Fig. 4 also comprises rods or wires 16A which are similar in shape and function to the rods or wires 16A of the hearing protectors 10 as shown in Figs. 1 to 3. The photovoltaic element 80 further comprises an edge region 80B (not visible because arranged underneath of the fixation frame 84). The solar cell 80A of the photovoltaic element 80 is kept in position by the cover 84A of the fixation frame 84, wherein the cover 84 covers the edge region 80B entirely and a small part of the solar cell 80A. As can be seen, the fixation frame 80 has an opening 84B formed or surrounded by the cover 84A such that the solar cell 80A of the photovoltaic element 80 is exposed to incident light. The headband 16 further comprises a support element 82, the details of which are not shown here, please see Fig. 3. The hearing protector 10 further comprises an electric cable 86 which is configured and arranged to transfer electric energy from the photovoltaic element 80 and/or data signals from and to the active components 40A, 40B of the earmuffs as well as to transfer electric energy from the photovoltaic element 80 to the battery 30 (not shown here) which may be arranged in one or both earmuffs 12, 14. As can be seen, the electric cable 86 is housed in the fixation frame 84 attached to the headband 16 of the hearing protector 10. As described above, the headband 16 with its rods or wires 16A are encapsulated or encompassed by the support element 82 and the fixation frame 84 with its cover 84A. As sealing 84C may be arranged between the cover 84A of the fixation frame 84 and the solar cell 80A of the photovoltaic element 80 such that ingress of moisture, dust and/or dirt is minimized or prevented. In the example shown, the sealing 84C is provided by the lower side of the cover 84A of the fixation frame 84, i.e. - in use - the side towards the user's head. A separate part representing a sealing is also conceivable although not shown here.

Fig. 5 shows in a schematic cross-sectional view a detail of the headband 16 of the hearing protector 10 as shown in Fig. 2. Attached to the headband 16 is a fixation frame 84 as described above. The fixation frame 84 with its cover 84A (not shown here, see Figs. 3 and 4) covers the edge region 80B of the photovoltaic element 80, wherein a sealing 84C is arranged therebetween to prevent ingress of moisture, dust and/or dirt as described above. The headband 16 further comprises rods or wires 16A configured and arranged to provide stability of the headband 16 on the one hand and to allow some flexibility or bending of the headband 16 on the other hand. The rods or wires 16A as shown here are similar in shape and function as the rods or wires 16A as shown in Figs. 1 to 4. The rods or wires 16A may be made of metal arranged in a curved shape and having a spring-like behavior. Fig. 5 further shows the electrical cable 86 for transfer of data and/or electric energy as described above, wherein the electric cable 86 is arranged at the headband 16, e.g. underneath of the photovoltaic element 80 as shown here. Alternatively, the electric cable 86 may be encompassed by the support element 82 and/or the fixation frame 84. Other alternatives are conceivable, e.g. the electric cable 86 may be arranged at one of the sides of the headband 16.

Fig. 6 shows in a schematic top view a detail of the honeycomb structure 88 of the support element 82 as shown in Fig. 3. The honeycomb structure 88 comprises ribs 88A, 88B which are arranged such that the ribs 88A are arranged in parallel to each other and perpendicular to the ribs 88B, which are in turn arranged in parallel to each other, and vice versa. Spaces or openings 88C are formed by the ribs 88A, 88B and therebetween, respectively. The ribs 88A, 88B form a pattern 88E. In the embodiment shown here, the ribs 88A, 88B are spaced evenly and the rib 88A is perpendicular to the rib 88B. Other configurations are conceivable, e.g. the ribs 88A, 88B may not be perpendicular to each other, but being arranged inclined or with an angle unequal to 90 degrees to each other. Also, it is conceivable that the spacing between the ribs 88A, 88B is not even, i.e. may vary over the extension of the pattern 88E of the ribs 88A, 88B in one or both directions thereof. Furthermore, the ribs 88A, 88B may be arranged such that these are not perpendicular to a base 88D (not visible here, please see Fig. 7), but arranged inclined with respect to the base 88D. Such an arrangement may be only partially or over the full extension of the pattern 88E formed by the ribs 88A, 88B in one or both directions thereof. In the example shown here, the openings or space 88C exhibit a rectangular cross-section seen from above. Other cross-sections are conceivable as well, e.g. square-shape, triangular, oval, round or circular or irregular or polygonal.

Fig. 7 shows in a schematic perspective view a detail of the honeycomb structure 88 of the support element 82 of the hearing protector 10 as shown in Fig. 3. As shown, the ribs 88A, 88B are arranged in the pattern 88E as kind of side-walls defining an opening or space 88C therebetween. The cross-section of the opening or space 88C is rectangular when seen from above, but other shapes are possible as explained for Fig. 6 above. In addition to what is shown in Figs. 3 and 6, the base 88D of the honeycomb structure 88 is shown here delimitating the opening or space 88C on the lower side thereof. The ribs 88A, 88B extend or protrude from the base 88D. The ribs 88A, 88B may be formed integral with the base 88D, e.g. by molding or extrusion. Alternatively, the ribs 88A, 88B may be attached to the base 88D by suitable attachment means incl. adhesive, molding them together or the like. This may provide for a combination of different materials for the ribs and the base, if required. It is noted that the honeycomb structure 88 of the support element 82 may not have a base 88D and may thus be open at both ends thereof.

## Claims

1. A hearing protector (10) comprising
∘ two earmuffs (12, 14) each comprising
▪ a cushion (22, 24),
▪ an outer shell (12A, 14A) defining a hollow space therein and
▪ sound absorbing material,
∘ a headband (16) for retaining the earmuffs (12, 14) in position when worn by a user (100),
wherein at least one of the earmuffs (12, 14) further comprises an active component (40A, 40B),
wherein the hearing protector (10) further comprises a photovoltaic element (80) for powering the active component (40A, 40B),
wherein the photovoltaic element (80) is attached to the headband (16) and wherein the photovoltaic element (80) is electrically connected to the active component (40A, 40B) of the earmuff (12, 14); **characterized in that** the photovoltaic element (80) is flexible.

2. The hearing protector (10) according to any of the preceding claims, wherein the photovoltaic element (80) is attached to the headband (16) by a fixation frame (84) and wherein the fixation frame (84) covers the entire perimeter of the photovoltaic element (80).

3. The hearing protector (10) according to claim 2, wherein the fixation frame (84) is a molded part attached to the headband (16) by overmolding.

4. The hearing protector (10) according to any of claims 2 or 3, wherein the fixation frame (84) comprises a sealing (84C) along the perimeter of the fixation frame (84).

5. The hearing protector (10) according to any of claims 2 to 4, wherein the fixation frame (84) covers not more than 12%, preferably not more than 10%, more preferred not more than 7,5% of the surface of the photovoltaic element (80).

6. The hearing protector (10) according to any one of the preceding claims, wherein the headband (16) comprises a support element (82) having a honeycomb structure (88).

7. The hearing protector (10) according to claim 6, wherein the honeycomb structure (88) exhibits an elongate shape and wherein the honeycomb structure (88) is preferably molded.

8. The hearing protector (10) according to any one of claim 6 or 7, wherein the honeycomb structure (88) comprises ribs (88A, 88B), preferably defining hollow spaces (88C) therebetween.

9. The hearing protector (10) according to any of the preceding claims, wherein the photovoltaic element (80) exhibits an elongated shape.

10. The hearing protector (10) according to any of the preceding claims further comprises a rechargeable battery (30), wherein the rechargeable battery is electrically connected to the photovoltaic element (80).

11. The hearing protector (10) according to any one of the preceding claims, wherein each earmuff comprises an active component (40A, 40B), the hearing protector (10) further comprising a signal cable for connecting the active components (40A, 40B) of each of the earmuffs (12, 14) and wherein the electric power for powering the active component (40A, 40B) is distributed over the signal cable (86)..

12. Method of retrofitting a hearing protector (10) comprising two earmuffs (12, 14) and a headband (16) for retaining the earmuffs (12, 14) in position when worn by a user (100) with an additional element, wherein at least one of the earmuffs (12, 14) further comprises an active component (40A, 40B),
the method comprising the steps of
- providing a hearing protector (10) comprising two earmuffs (12, 14) and a headband (16) for retaining the earmuffs (12, 14) in position when worn by a user (100), wherein at least one of the earmuffs (12, 14) comprises an active component (40A, 40B)
- providing an additional element, and
- attaching the additional element to the headband (16),
wherein the earmuffs (12, 14) each comprise
i. a cushion (22, 24),
ii. an outer shell (12A, 14A) defining a hollow space therein and
iii. sound absorbing material;
**characterized in that**
the additional element in the steps of providing and attaching the additional element is an elongate photovoltaic element (80) that is flexible, and that the method further comprises:
- electrically connecting the photovoltaic element (80) to the active component (40A, 40B) of the hearing protector (10),

13. The method according to claim 12, wherein the headband (16) comprises a support element (82) having a honeycomb structure (88).

14. The method according to claim 13, wherein the honeycomb structure (88) exhibits an elongate shape and wherein the honeycomb structure (88) is preferably molded.

15. The method according to any one of claims 13 or 14, wherein the honeycomb structure (88) comprises ribs (88A, 88B), preferably defining hollow spaces (88C) formed therebetween.

## Patentansprüche

1. Ein Gehörschutz (10), aufweisend
∘ zwei Ohrmuscheln (12, 14), jeweils aufweisend
▪ ein Polster (22, 24),
▪ eine Außenschale (12A, 14A), die einen Hohlraum darin definiert, und
▪ klangabsorbierendes Material,
∘ ein Kopfband (16) zum Inpositionhalten der Ohrmuscheln (12, 14), wenn sie durch einen Benutzer (100) getragen werden,
wobei mindestens eine der Ohrmuscheln (12, 14) ferner eine aktive Komponente (40A, 40B) aufweist,
wobei der Gehörschutz (10) ferner ein Photovoltaikelement (80) zum Antreiben der aktiven Komponente (40A, 40B) aufweist,
wobei das Photovoltaikelement (80) an dem Kopfband (16) befestigt ist und wobei das Photovoltaikelement (80) mit der aktiven Komponente (40A, 40B) der Ohrmuschel (12, 14) elektrisch verbunden ist; **dadurch gekennzeichnet, dass** das Photovoltaikelement (80) flexibel ist.

2. Der Gehörschutz (10) nach einem der vorstehenden Ansprüche, wobei das Photovoltaikelement (80) an dem Kopfband (16) durch einen Fixierungsrahmen (84) befestigt ist und wobei der Fixierungsrahmen (84) den gesamten Umfang des Photovoltaikelements (80) abdeckt.

3. Der Gehörschutz (10) nach Anspruch 2, wobei der Fixierungsrahmen (84) ein Formteil ist, das an dem Kopfband (16) durch ein Überspritzen befestigt ist.

4. Der Gehörschutz (10) nach einem der Ansprüche 2 oder 3, wobei der Fixierungsrahmen (84) eine Dichtung (84C) entlang des Umfangs des Fixierungsrahmens (84) aufweist.

5. Der Gehörschutz (10) nach einem der Ansprüche 2 bis 4, wobei der Fixierungsrahmen (84) nicht mehr als 12 %, vorzugsweise nicht mehr als 10 %, mehr bevorzugt nicht mehr als 7,5 %, der Oberfläche des Photovoltaikelements (80) abdeckt.

6. Der Gehörschutz (10) nach einem der vorstehenden Ansprüche, wobei das Kopfband (16) ein Stützelement (82) aufweist, das eine Wabenstruktur (88) hat.

7. Der Gehörschutz (10) nach Anspruch 6, wobei die Wabenstruktur (88) eine längliche Form vorweist und wobei die Wabenstruktur (88) vorzugsweise geformt ist.

8. Der Gehörschutz (10) nach einem von Anspruch 6 oder 7, wobei die Wabenstruktur (88) Rippen (88A, 88B) aufweist, die vorzugsweise Hohlräume (88C) dazwischen definieren.

9. Der Gehörschutz (10) nach einem der vorstehenden Ansprüche, wobei das Photovoltaikelement (80) eine längliche Form vorweist.

10. Der Gehörschutz (10) nach einem der vorstehenden Ansprüche weist ferner eine wiederaufladbare Batterie (30) auf, wobei die wiederaufladbare Batterie mit dem Photovoltaikelement (80) elektrisch verbunden ist.

11. Der Gehörschutz (10) nach einem der vorstehenden Ansprüche, wobei jede Ohrmuschel eine aktive Komponente (40A, 40B) aufweist, der Gehörschutz (10) ferner aufweisend ein Signalkabel zum Verbinden der aktiven Komponenten (40A, 40B) jeder der Ohrmuscheln (12, 14) und wobei die elektrische Energie zum Antreiben der aktiven Komponente (40A, 40B) über das Signalkabel (86) verteilt ist..

12. Verfahren zum Nachrüsten eines Gehörschutzes (10), aufweisend zwei Ohrmuscheln (12, 14) und ein Kopfband (16) zum Inpositionhalten der Ohrmuscheln (12, 14), wenn sie durch einen Benutzer (100) getragen werden, mit einem zusätzlichen Element, wobei mindestens eine der Ohrmuscheln (12, 14) ferner eine aktive Komponente (40A, 40B) aufweist,
das Verfahren aufweisend die Schritte
- Bereitstellen eines Gehörschutzes (10), aufweisend zwei Ohrmuscheln (12, 14) und ein Kopfband (16) zum Inpositionhalten der Ohrmuscheln (12, 14), wenn sie durch einen Benutzer (100) getragen werden, wobei mindestens eine der Ohrmuscheln (12, 14) eine aktive Komponente (40A, 40B) aufweist
- Bereitstellen eines zusätzlichen Elements und
- Befestigen des zusätzlichen Elements an dem Kopfband (16), wobei die Ohrmuscheln (12, 14) jeweils aufweisen
i. ein Polster (22, 24),
ii. eine Außenschale (12A, 14A), die einen Hohlraum darin definiert, und
iii. klangabsorbierendes Material;
**dadurch gekennzeichnet, dass**
das zusätzliche Element in den Schritten des Bereitstellens und des Befestigens des zusätzlichen Elements ein längliches Photovoltaikelement (80) ist, das flexibel ist, und dass das Verfahren ferner aufweist:
- elektrisches Verbinden des Photovoltaikelements (80) mit der aktiven Komponente (40A, 40B) des Gehörschutzes (10),

13. Das Verfahren nach Anspruch 12, wobei das Kopfband (16) ein Stützelement (82) aufweist, das eine Wabenstruktur (88) hat.

14. Das Verfahren nach Anspruch 13, wobei die Wabenstruktur (88) eine längliche Form vorweist und wobei die Wabenstruktur (88) vorzugsweise geformt ist.

15. Das Verfahren nach einem der Ansprüche 13 oder 14, wobei die Wabenstruktur (88) Rippen (88A, 88B) aufweist, die vorzugsweise Hohlräume (88C) definieren, die dazwischen ausgebildet sind.

## Revendications

1. Protecteur auditif (10) comprenant
∘ deux cache-oreilles (12, 14) comprenant chacun
■ un coussinet (22, 24),
■ une enveloppe externe (12A, 14A) définissant un espace creux à l'intérieur de celle-ci et
■ un matériau absorbant acoustique,
∘ un serre-tête (16) permettant de retenir les cache-oreilles (12, 14) en position lorsqu'ils sont portés par un utilisateur (100),
dans lequel au moins l'un des cache-oreilles (12, 14) comprend en outre un composant actif (40A, 40B),
dans lequel le protecteur auditif (10) comprend en outre un élément photovoltaïque (80) destiné à alimenter le composant actif (40A, 40B),
dans lequel l'élément photovoltaïque (80) est assujetti au serre-tête (16) et dans lequel l'élément photovoltaïque (80) est connecté de manière électrique au composant actif (40A, 40B) du cache-oreilles (12, 14) ; **caractérisé en ce que** l'élément photovoltaïque (80) est flexible.

2. Protecteur auditif (10) selon l'une quelconque des revendications précédentes, dans lequel l'élément photovoltaïque (80) est assujetti au serre-tête (16) par un cadre de fixation (84) et dans lequel le cadre de fixation (84) couvre tout l'intégralité du périmètre de l'élément photovoltaïque (80).

3. Protecteur auditif (10) selon la revendication 2, dans lequel le cadre de fixation (84) est une pièce moulée assujettie au serre-tête (16) par surmoulage.

4. Protecteur auditif (10) selon l'une quelconque des revendications 2 ou 3, dans lequel le cadre de fixation (84) comprend un joint d'étanchéité (84C) le long du périmètre du cadre de fixation (84).

5. Protecteur auditif (10) selon l'une quelconque des revendications 2 à 4, dans lequel le cadre de fixation (84) ne couvre pas plus de 12 %, de préférence pas plus de 10 %, plus préférablement pas plus de 7,5 % de la surface de l'élément photovoltaïque (80).

6. Protecteur auditif (10) selon l'une quelconque des revendications précédentes, dans lequel le serre-tête (16) comprend un élément de support (82) ayant une structure en nid d'abeilles (88).

7. Protecteur auditif (10) selon la revendication 6, dans lequel la structure en nid d'abeilles (88) présente une forme allongée et dans lequel la structure en nid d'abeilles (88) est de préférence moulée.

8. Protecteur auditif (10) selon l'une quelconque des revendications 6 ou 7, dans lequel la structure en nid d'abeilles (88) comprend des nervures (88A, 88B), définissant de préférence des espaces creux (88C) entre elles.

9. Protecteur auditif (10) selon l'une quelconque des revendications précédentes, dans lequel l'élément photovoltaïque (80) présente une forme allongée.

10. Protecteur auditif (10) selon l'une quelconque des revendications précédentes comprend en outre une batterie rechargeable (30), dans lequel la batterie rechargeable est connectée de manière électrique à l'élément photovoltaïque (80).

11. Protecteur auditif (10) selon l'une quelconque des revendications précédentes, dans lequel chaque cache-oreilles comprend un composant actif (40A, 40B), le protecteur auditif (10) comprenant en outre un câble de signal destiné à connecter les composants actifs (40A, 40B) de chacun des cache-oreilles (12, 14) et dans lequel l'alimentation électrique destinée à alimenter le composant actif (40A, 40B) est distribuée sur le câble de signal (86).

12. Procédé de mise à niveau d'un protecteur auditif (10) comprenant deux cache-oreilles (12, 14) et un serre-tête (16) destiné à retenir les cache-oreilles (12, 14) en position lorsqu'ils sont portés par un utilisateur (100) avec un élément supplémentaire, dans lequel au moins l'un des cache-oreilles (12, 14) comprend en outre un composant actif (40A, 40B),
le procédé comprenant les étapes consistant à
- fournir un protecteur auditif (10) comprenant deux cache-oreilles (12, 14) et un serre-tête (16) destiné à retenir les cache-oreilles (12, 14) en position lorsqu'ils sont portés par un utilisateur (100), dans lequel au moins l'un des cache-oreilles (12, 14) comprend un composant actif (40A, 40B)
- fournir un élément supplémentaire, et
- assujettir l'élément supplémentaire au serre-tête (16),
dans lequel les cache-oreilles (12, 14) comprennent chacun
i. un coussinet (22, 24),
ii. une enveloppe externe (12A, 14A) définissant un espace creux à l'intérieur de celle-ci et
iii. un matériau absorbant acoustique ;
**caractérisé en ce que**
l'élément supplémentaire dans les étapes consistant à fournir et à assujettir l'élément supplémentaire est un élément photovoltaïque allongé (80) qui est flexible, et **en ce que** le procédé comprend en outre :
- la connexion de manière électrique de l'élément photovoltaïque (80) au composant actif (40A, 40B) du protecteur auditif (10),

13. Procédé selon la revendication 12, dans lequel le serre-tête (16) comprend un élément de support (82) ayant une structure en nid d'abeilles (88).

14. Procédé selon la revendication 13, dans lequel la structure en nid d'abeilles (88) présente une forme allongée et dans lequel la structure en nid d'abeilles (88) est de préférence moulée.

15. Procédé selon l'une quelconque des revendications 13 ou 14, dans lequel la structure en nid d'abeilles (88) comprend des nervures (88A, 88B), définissant de préférence des espaces creux (88C) formés entre elles.
